# EUROPEAN PATENT APPLICATION

(11) **EP 0 604 022 A1**
(43) Date of publication of application: **29.06.1994**
(21) Application number: 93309386.6
(22) Date of filing: 24.11.1993
(51) Int. Cl.: A61F 2/06, A61L 31/00

(54) **Multilayered biodegradable stent and method for its manufacture**

(30) Priority: 22.12.1992 US 995579
(71) Applicant: ADVANCED CARDIOVASCULAR SYSTEMS, INC., Santa Clara California 95052 (US)
(72) Inventor: Eury, Robert P., Cupertino, California 95014 (US)
(74) Representative: Baverstock, Michael George Douglas

(57) **Abstract**

A stent (12) of multilayered luminated construction wherein one layer (18) addresses the structural requirements of the stent and additional layers (20,22) release drugs at predictable rates. Both the structural layer (18) as well as the drug releasing layers (20,22) are eventually completely resorbed by the body.

## Description

The present invention relates to expandable intraluminal vascular grafts, generally referred to as stents. More particularly, the invention pertains to stents that are biodegradable and capable of releasing therapeutic drugs.

Stents are implanted within lumens of vessels of the body in order to maintain the patency of such vessels. A variety of delivery systems have been devised that facilitate the placement and deployment of stents. The stent initially is manipulated while in its contracted state, wherein its reduced diameter more readily allows it to be introduced into the lumen and maneuvered into place. Once in place, it is enlarged to a diameter either greater than or equal to the diameter of the lumen so as to allow the free flow of fluids therethrough.

A system especially adapted for coronary applications employs a stent design that incorporates a combination of interacting elements which serve to automatically lock the stent into its enlarged configuration upon expansion. The stent is moved into position along a guidewire that previously has been placed in the lumen where the stent is to be deployed. Inflation of a balloon about which the stent rests causes the stent to expand and lock. Subsequent deflation of the balloon and withdrawal of the catheter and guidewire leaves the stent in place. Elements extending from the surface of the stent engage the vessel walls to positively maintain the stent in position within the lumen.

Stents heretofore typically have been formed of non-toxic, substantially biocompatible metals such as stainless steel, tantalum, or gold. However, it has been determined that typically within about seven to twenty-one days the endothelial layer of the artery or vessel grows into and throughout the walls of the stent, at which point the utility of the stent substantially is diminished and the continued presence of the stent in the lumen may cause any of a variety of problems or complications. Therefore, it has been proposed to form stents of biodegradable or bioabsorbable materials that are completely resorbed by the body within a period of time.

Continued pharmacological treatment of the vessel or condition that made the implantation of the stent necessary often is required or desirable. Such treatment typically is most effective when administered locally and, as a result, it has been suggested to rely on the stent for the delivery of drugs to provide this treatment. Materials are known that are capable of absorbing certain drugs and subsequently releasing the drugs at a substantially predictable rate for a proscribable period of time under particular environmental conditions. By forming the stent of such drug-impregnated material or by otherwise associating such material with the stent, the stent can achieve the dual purpose of maintaining patency and of dispensing drugs.

The prior art has been unable to provide a stent that is completely absorbed by the body after deployment, possesses the physical properties necessary to facilitate its implantation, performs a primary function of maintaining the patency of the vessel for a period of time, and gradually releases a drug prior to its resorption. Previous attempts to impart the necessary physical properties to drug-releasing materials or attempts to impart such properties to those materials without compromising the drug-releasing properties or the efficacy of the drugs absorbed have been unsuccessful.

The present invention provides a stent that is both completely resorbable by the body and capable of delivering certain drugs. Moreover, the stent possesses all the physical properties necessary for it to perform its structural function as well as to facilitate its implantation. This is achieved by employing a multilayered, laminated construction. A first bioabsorbable layer is selected for its physical properties. One or more additional resorbable layers are selected for the ability of the layer or layers to retain various drugs and then gradually release the drugs upon exposure to the particular environment to which the stent is exposed to upon implantation.

The laminated construction allows the combination in a single stent of a plurality of different drug containing materials. By appropriate configuration of the layers, drugs can be either delivered simultaneously with deployment of the stent or released in a predetermined and controlled sequence. Moreover, different parts of the anatomy can be targeted for treatment using different drugs. That is, a drug-containing layer which is associated only with the exterior surface of the stent would cause that drug to be released directly into the vessel wall while a drug-containing layer associated only with the interior surface of the stent would cause the drug to be released into, and to free flow within, the lumen.

The laminated construction of the stent allows the structural layer to be fabricated before it is laminated. Therefore, any layer can be subjected to rigorous conditioning during its processing and treatment that might be advantageous in order to impart sufficient strength to the layer or layers. In the case of prior art stents, this rigorous conditioning might have the effect of deteriorating or causing a degradation in the effect of any drug or drug-releasing materials. The present invention provides for the drug-impregnated materials to be combined with the structural layer only after the fabrication of the structured layer is complete.

The material selected for the structural layer of the stent of the present invention must be reabsorbable while providing the necessary physical characteristics. These requirements can be satisfied by using polymers such as poly-L-lactic acid or polyglycolic acid that have been extruded and oriented to obtain maximum tensile strength and optimal flexural properties.

The drug-releasing layers are selected based on the properties of the materials used to retain sufficient quantities of particular drugs, to release those drugs at a constant or at least predictable rate when exposed to the environment encountered upon implantation, and to eventually become completely absorbed by the body. Polymers capable of such functions include poly-DL-lactic acid or polycaprolactone. Such polymers are first intermixed with the drug or drugs to be delivered and then are either extruded or solvent cast. The drug-containing layer or layers and the structural layer subsequently are laminated to one another using heat or solvents.

The present invention advantageously is applied to stents implantable in a coronary artery after an angioplasty procedure has been performed wherein the exterior surface of the stent releases a drug into the vessel wall that tends to discourage restenosis and also discourages coagulation of the fluid passing through the lumen. Alternatively, a stent according to the present invention may be utilized to treat prostate cancer. In this application, a chemotherapeutic drug is released directly into the urethra via the implanted stent.

These and other features and advantages of the present invention will become apparent from the following detailed description of the preferred embodiments which, taken in conjunction with the accompanying drawings, illustrates by way of example the principles of the invention.

In the drawings:
Figure 1 is a perspective view of a stent of the present invention; and
Figure 2 is an enlarged cross sectional view showing the laminated construction of the stent of Figure 1.

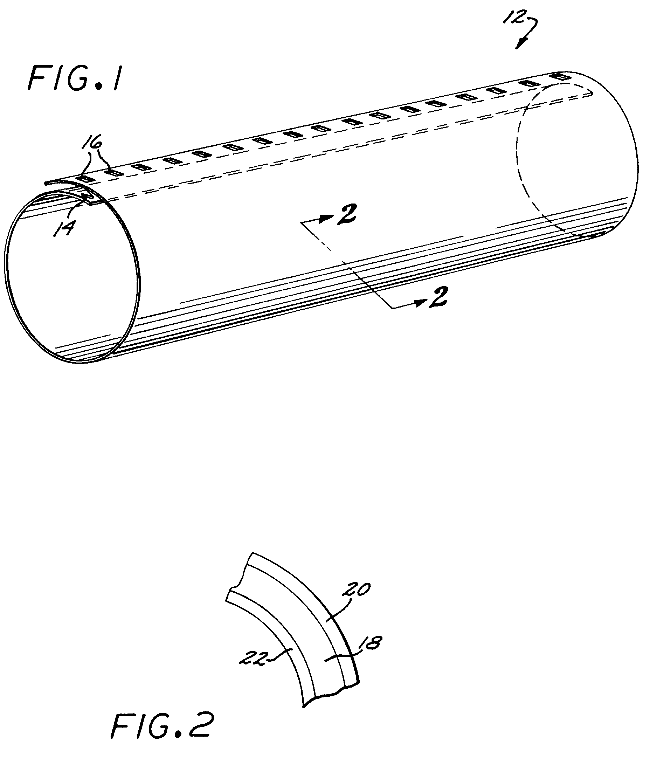

The Figures illustrate a preferred embodiment of the present invention. Generally, Figure 1 illustrates stent 12 prior to implantation. The stent is formed as a furled cylinder of sufficiently small outer diameter so as to be transportable through the lumen in which it is to be deployed and of sufficiently large internal diameter to receive a balloon catheter therein. The tabs 14 extending from the outer surface of the catheter are sized to engage apertures 16 after the balloon has been inflated. The process of inflating the balloon causes the cylinder to unfurl and thereby expand. Once elements 14 and 16 have engaged, the stent effectively is locked into its expanded state and cannot recontract.

Figure 2 is an enlarged crosssectional view of the stent 12 according to the present invention. The stent comprises a laminated structure comprising multiple layers. The particular embodiment illustrated has three layers, layers 18, 20, and 22. Central, relatively thick layer 18 comprises the structural component of the stent which imparts the necessary physical characteristics to make the stent capable of maintaining the patency of a lumen. This central layer also imparts the desired flexural characteristics to the stent to allow it to be positioned as well as to be expanded once positioned. Thinner layers on either side of structural layer 18 deliver pharmacological agents. Materials that form the thinner layers are selected for the ability of the materials to absorb drugs and subsequently release the drugs at predictable rates once the stent is subjected to the environment encountered upon implantation. In the embodiment illustrated, the layers are disposed such that each is adjacent a surface of central structural layer 18. These layers may contain the same type of drug or different drugs. Alternatively, only one drug-releasing layer might be laminated to one surface of the stent. In still another aspect, additional drug-releasing layers can be built up on top of one another to allow sequential release of various medicaments.

The material employed for the structural layer is selected based on the ability of the material to impart the necessary physical properties to the stent as well for its capacity to be completely reabsorbed by the body. "Resorbable" is meant to encompass all those materials that are either biodegradable, bioerodable, or bioabsorbable and includes materials that break down over time and are gradually absorbed or those that are eliminated by the body regardless of whether the degradation mainly is due to hydrolysis or is mediated by metabolic processes. As previously mentioned, the strength of such material must be such that once the stent is in its expanded and locked form, it is capable of maintaining the patency of the vessel into which it is implanted. Additionally, the physical characteristics of the material of the structural layer must be such as to provide flexibility sufficient to allow it to be expanded by, for example, the inflation of a balloon contained therein. Furthermore, a degree of longitudinal flexibility is desirable in order to facilitate transportation of the stent through a potentially tortuous path through the lumen to its intended implantation site.

Materials with the capacity to provide both the structural integrity and resorbability required being resorbable typically are polymeric in nature. Polymers such as poly-L-lactic acid or polyglycolic acid which have been extruded and oriented by known methods to obtain maximum tensile strength as well as optimal flexural properties are well-suited for such application. Polyorthoesters or polyanhydrides also could be used. In the present invention, the laminated construction of the stent allows the structural layer to be processed and treated to enhance its physical properties without concern for the effect such potentially rigorous conditions would have on drug and drug-containing materials.

The materials used for drug-releasing layers 20 and 22 are selected for the ability to be absorbed and the ability to retain various drugs and subsequently release the medicaments at a predictable rate upon implantation of the stent. Materials found to be especially advantageous for such purposes include polymers such as poly-DL-lactic acid and polycaprolactone. These polymers can be intermixed with the drug or drugs to be released and subsequently extruded or solvent cast by well-known methods.

After the central structural layer and any drug-releasing layers have been fabricated, the layers are laminated to one another using heat or solvents. For example, a layer of poly-L-lactic acid and a poly-DL-lactic acid are combinable by configuring the two layers in intimate contact and subjecting the layers to a temperature of about 55°C. The completed laminate subsequently is stamped or laser-cut to the appropriate dimensions. The elements and features necessary to initially maintain the stent in a furled state and subsequently allow it to be locked into its expanded state are formed by similarly well known methods. A final furling or shaping operation renders the stent substantially ready for use.

The ultimate purpose of the stent dictates the dimensions of the stent, the strength requirements and physical characteristics of the stent, and the particular drugs and drug delivery rates selected. For example, when the goal is to maintain the patency of a coronary artery, a stent according to the present invention wherein the innermost layer exposed to the lumen is configured to release a drug that reduces the likelihood of thrombosis. Heparin or prostacyclin are among the drugs appropriate for this purpose. In such an application, outer layer 20 could advantageously release drugs that address restenosis. Drugs that have been found to be effective for this purpose include angiopeptin, methotrexate, and heparin.

While a particular form of the invention has been illustrated and described, it also will be apparent to those skilled in the art that various modifications can be made without departing from the spirit and scope of the invention. Any of a variety of stent designs and applications can benefit from the present invention. Accordingly, it is not intended that the invention be limited except by the appended claims.

## Claims

1. An expandable intraluminal stent for implantation in a vessel, comprising:
a first layer formed of resorbable material selected to impart structural rigidity to said stent; and
a second layer of a resorbable material, joined to said first layer and selected to release a therapeutic drug at a selected rate therefrom, whereby upon implantation, said stent initially provides a desired degree of structural support to said vessel, releases said drug at the selected rate and eventually is completely resorbed.

2. A stent as claimed in claim 1, wherein said first layer is further selected to impart additional desired physical characteristics to said stent.

3. A stent as claimed in claim 1, wherein said first layer comprises poly-L-lactic acid, or polyglycolic acid.

4. A stent as claimed in any one of claims 1 to 3, wherein said second layer comprises poly-DL-lactic acid or polycaprolactone.

5. A stent as claimed in any one of the preceding claims wherein said stent is implantable in a lumen upstream from a cancerous growth and said second layer is selected to release a chemotherapeutic drug.

6. A stent as claimed in any one of the preceding claims wherein said second layer is selected to release heparin.

7. A stent as claimed in any one of the preceding claims wherein a third layer of resorbable material is joined to said first layer and selected to release a therapeutic drug at a selected rate therefrom, whereby upon implantation, said stent initially provides a desired degree of structural support to said vessel, releases said drugs at the selected rates and eventually is completely resorbed.

8. A stent as claimed in claim 7, wherein the drugs released by said first and second layer are identical.

9. A stent as claimed in claim 7, wherein the drugs released by said first and second layer are different.

10. A stent as claimed in claim 9, wherein said stent is implantable within a coronary artery, said second layer is joined to the outer side of said stent's first layer and releases a drug that addresses restenosis and said third layer is joined to the inner side of said stent's first layer and releases a drug that addresses thrombosis.

11. A stent as claimed in claim 7, wherein said second layer is selected to release angiopeptin and said third layer is selected to release heparin.

12. A method of fabricating a biodegradable, drug releasing stent for implantation within a lumen, comprising the steps of:
forming a first layer of biodegradable material having physical properties necessary to enable a stent to perform its structural function within said lumen;
forming a second layer of biodegradable material which releases a selected drug upon exposure to an environment such as is encountered within said lumen; and
joining said second layer to said first layer to provide a laminated structure that is biodegradable, drug releasing and has the physical properties necessary for said stent to perform its structural function within said lumen.

13. A method as claimed in claim 12, further comprising the steps of:
forming a third layer of biodegradable material which releases a selected drug upon exposure to an environment such as is encountered within said lumen; and
joining said third layer to said first layer.

14. A method as claimed in claim 12 or claim 13, wherein said first layer is formed by extrusion and orientation of a polymeric material.

15. A method as claimed in claim 14, wherein said polymeric material comprises poly-L-lactic acid or polyglycolic acid.

16. A method as claimed in any one of claims 12 to 15, wherein said second layer comprises a mixture of said drug and a polymeric material.

17. A method as claimed in claim 16, wherein said polymeric material comprises poly-DL-lactic acid or a polycaprolactone.

18. A method as claimed in any one of claims 12 to 17, wherein said second layer is joined to said first layer with the application of heat or with the use of solvents.

19. A method as claimed in any one of claims 13 to 17, wherein said second and third layers are joined to said first layer with the application of heat or with the use of solvents.
